Europäisches Patentamt

European Patent Office

Office européen des brevets

(19)

(11) Publication number: **0 160 500 B1**

# EUROPEAN PATENT SPECIFICATION

(12)

(45) Date of publication of patent specification: **24.07.91**

(51) Int. Cl.⁵: **A61F 13/10, A61B 17/54**

(21) Application number: **85302843.9**

(22) Date of filing: **24.04.85**

(54) Temporary cover to foster cosmeticcally pleasing healing of nail bed tissue.

(30) Priority: **30.04.84 US 605112**

(43) Date of publication of application:
**06.11.85 Bulletin 85/45**

(45) Publication of the grant of the patent:
**24.07.91 Bulletin 91/30**

(84) Designated Contracting States:
**AT BE CH DE FR GB IT LI NL SE**

(56) References cited:
**EP-A- 0 099 635**
**EP-A- 0 171 134**
**DE-C- 898 072**
**FR-A- 1 424 886**
**US-A- 3 277 900**

(73) Proprietor: **Ogunro, E. Olayinka**
**625 Ray Avenue**
**Desoto Texas 75115(US)**

(72) Inventor: **Ogunro, E. Olayinka**
**625 Ray Avenue**
**Desoto Texas 75115(US)**

(74) Representative: **Goodman, Christopher et al**
**Eric Potter & Clarkson St. Mary's Court St.**
**Mary's Gate**
**Nottingham NG1 1LE(GB)**

## Description

This invention relates generally to a temporary cover useful in dealing with the results of diseases and/or injuries to the tips of a person's digits--on either the hand or foot. More specifically, it relates to a cover having utility to foster healing of the ends of a person's digits, in order that nail plates (i.e. fingernails and toenails) can be restored to a condition that more nearly approaches their original condition.

It is well known that injuries to the ends of a person's digits occur frequently, and those injuries are often more than simply painful. That is, when a weekend carpenter strikes at a steel nail and hits his thumb instead, he may experience a pain that lasts for several minutes. But if he mishandles an electric saw and cuts himself, the initial pain may be only a small part of his overall problem--because a rotating saw blade that cuts through the top of a fingernail can cause damage that is not easily taken care of by the body's natural healing processes. Typically, when a person's nail plate has been cut off or it is so badly damaged that it must be surgically removed, the nail bed has been permitted to heal without the presence of any restraint. The result is typically an uneven and shrunken region of "scarry" tissue where there had once been a soft, smooth cushion of healthy tissue. Furthermore, the soft gauze dressings that are typically applied to a healing nail bed often become stuck to the healing tissue when blood or other body liquids dry in contact with the dressing. When a doctor later removes those dressings, the pain is often extreme-because an unprotected nail bed is one of the most sensitive spots on a person's body.

After a nail bed has healed, the nail plate will often grow back over the nail bed, assuming that at least some of the nail's root was not lost as a result of the injury. If the healed bed is not smooth and "natural", the nail that grows over that bed will be similarly deformed, and an irregular or split--and usually unpleasing--nail is the result. Examples of such deformed nails are illustrated at page 250 of an article by Zook, Guy and Russell entitled "A study of nail bed injuries: Causes, treatment and prognosis" that appeared in the March, 1984 issue of The Journal of Hand Surgery.

Attention has been given in recent years to cosmetic or reconstructive surgery for the purpose of replacing a damaged or disfigured nail with a prosthetic nail. And, two such examples of prosthetic nails are revealed in U.S. patents issued to this inventor, namely, No. Des. 265,507 entitled "Prosthetic Nail" which issued July 20, 1982 and No. 4,445,234 (EP-A-0099635 being a patent family member) entitled "Prosthetic Nail" which issued May 1, 1984. No matter how beneficial these prosthetic nails may be for solving the cosmetic and functional problems of a missing nail, it would be preferable to foster the rehabilitation of a person's fingertips or toes without being forced to use a nail prothesis. And, it has now been concluded that the healing of a nail bed can be fostered by temporarily placing a particular kind of cover or splint over a damaged nail bed during the critical time that it is initially recovering from an injury. Such a cover must be smooth and in intimate contact with the nail bed and provide a sufficient restraint as to force the healing tissue to remain in an essentially normal condition, i.e., without irregular collagen. Such a temporary cover is disclosed in EP-A-0171134 which falls under Article 54(3) EPG. When the cover/splint of this invention is subsequently removed after a few weeks, the resulting nail that eventually grows back over the healed bed will appear much more smooth and will be less distinguishable from the person's other, non-injured nails. This disclosure, therefore, is intended to present the details of a particular structure and the method of utilizing that structure--so as to enable a person skilled in the art to practice the invention.

Besides the cosmetic benefit that will be realized from use of the disclosed split, an additional benefit will be realized from the physical protection of a tender area, especially during the first few days after a finger or toe has been damaged. With a temporary splint covering a freshly exposed nail bed, the risk of unplanned contact with nail bed tissue can be eliminated; and, any "point" pressure from contact with a pencil or the like can be distributed by a relatively firm splint so as to produce broader "area" pressure. It is another object of this invention, therefore, to protect a patient against unnecessary pain during those first several days after the injury.

The goal of minimizing pain when a patient tries to resume normal activities may seem like an adequate goal in and of itself, but another benefit from the use of the splint disclosed herein is a reduction in the amount of pain-killing drugs that the patient might otherwise feel is either necessary or desirable. And, it is now being more widely appreciated that pain-killing drugs should be used with the recognition that chemical dependency is a major threat to the full recovery of a patient. It should be appreciated, therefore, that the reduction of pain associated with a healing digit has both simple and complicated facets; and any structure which contributes to pain reduction is therefore doubly useful.

Accordingly, the temporary cover of the invention is characterised as in claim 1.

With the cover of the invention, when the nail plate grows back over the healed bed, it will be smooth and will look normal.

Reference is made to the drawings, in which:

Figure 1 is a perspective view of a splint in accordance with this invention, showing the generally concave underside of the splint;

Figure 2 is a side elevational view of the splint (drawn at a different scale), showing the curvature in a longitudinal plane;

Figure 3 is a front elevational view (which could also be called an end view) of the splint, on the same scale as Figure 2;

Figure 4 is a side elevational view of another embodiment of the invention, clearly showing a non-linear edge along the visible side; and

Figure 5 is a top plan view of an embodiment of the invention having "bleed" holes therein.

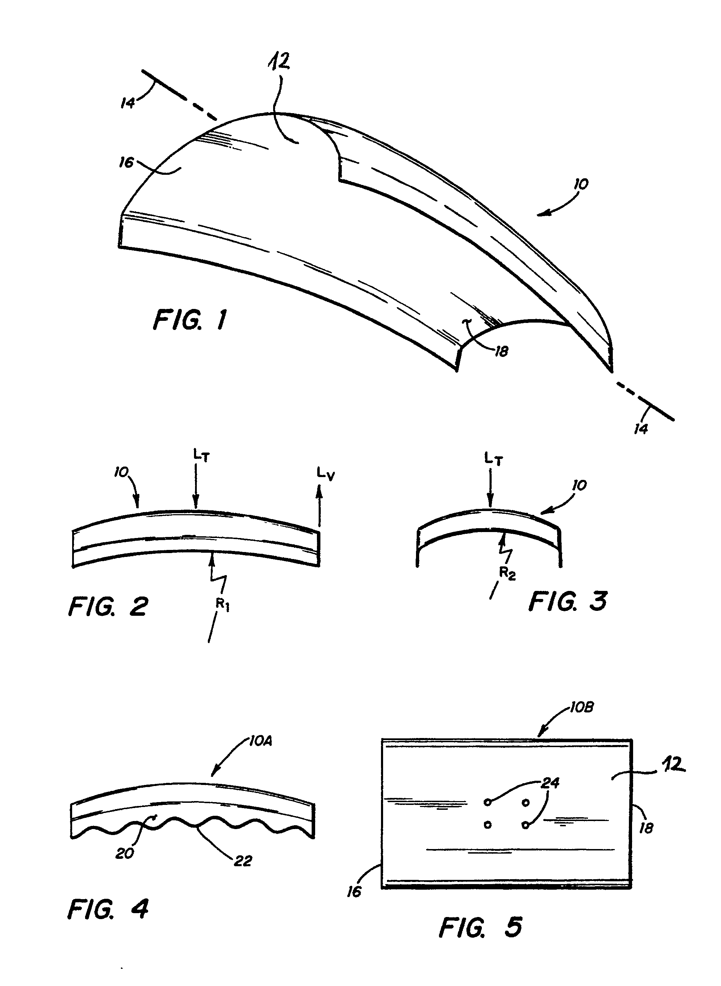

Referring initially to Figures 1-3, the temporary cover or splint for use on the digits of a person's hand or foot is shown in its simplest configuration. The cover or splint 10 includes a generally elongate sheet 12 of firm but resilient material which is inert to body fluids and is water impervious. A preferred material for the sheet 12 is a polypropylene sold by Hercules Incorporated of Wilmington, Delaware under the trademark "Profax 6523". The sheet 12 has a compound curvature so that it approximates the natural shape of a human nail plate. That is, the sheet has a first radius $R_1$ which defines a curve in a longitudinal plane; this first radius will usually be on the order of 1.5 inches (38 mm). The sheet 12 also has a second radius $R_2$ which defines a curve in a transverse plane; this second radius is substantially smaller than $R_1$, usually about 1/3 thereof. The preferred transverse radius $R_2$ is about 0.4 inch (10 mm), and the sheet 12 obviously appears somewhat like a segment of a cylinder when viewed along its longitudinal axis 14. The width of the sheet 12 as viewed along said longitudinal axis corresponds to a segment of a circle having an included angle of about 70 degrees.

The sheet's forward (distal) end 16 is preferably shaped the same as its proximal end. This offers an advantage to the surgeon in that there is no criticality as to which end is inserted into the eponychial fold. Besides having no "right" and "wrong" ends when the splint 10 is removed from a sterile package by the surgeon, it also will often be slightly long for most fingernails and toenails. A preferred length of about 1 inch (25 mm) permits some tolerance for error as the surgeon tries to match the shape of the injured digit--by trimming off small segments of the sheet. As long as the surgeon works by cutting away only small bits at a time, he might even be able to cut off and discard his first effort at shaping the distal end, while still leaving enough material in the sheet to start again with his shaping work--at either end of the symmetrical sheet.

The sheet's proximal end 18, both as manufactured and as installed, will preferably be relatively "square", in the sense that it will have two corners of approximately 90 degrees each. In this respect, the splint 10 differs significantly from previously known appendages for nails--which are typically oval or elliptical in shape. Also, the thickness of one proximal end 16 is ideally the same thickness as the rest of the sheet 12, and should be at least thick enough to provide enough "body" to restrain or support the eponychial fold. This is important because a characteristic of the body's natural healing process is to eliminate the space between the eponychial fold and the nail bed, if some substantive material is not positioned within that space so as to keep it from closing. The "square" corners of the sheet 12 also inhibit the fold from contracting (in a transverse direction), which would cause the size of a healed nail bed to be unnecessarily reduced.

A preferred thickness for a sheet 12 is within the range of about 0.015-0.020 inch (0.38 to 0.5 mm). A splint 10 having the shape illustrated in Figure 1 and having the preferred dimensions (e.g., thickness of 0.020 inch (0.5 mm), $R_1$ of 1.5 inches (38 mm), $R_2$ of 0.4 inch (10 mm)) will have a surprisingly strong resistance to vertical loading in the direction indicated by the arrow $L_v$ in Figure 2. In fact, it has been determined that a vertical lifting load $L_v$ of over five pounds can be imposed in the distal end of the splint 10 (when it is anchored at its proximal end) without causing the splint to bend. This is particularly advantageous because accidental loading of the splint 10 in the direction indicated by force $L_v$ can be imposed on a splint when the patient reaches for a cup, drawer, door latch, etc., and inadvertently strikes an immovable object. It is important, therefore, that the splint 10 have sufficient rigidity--based upon its geometry--to provide true protection during the critical healing period.

It may be instructive at this time to compare the splint 10 as described herein with previously disclosed attempts to providing temporary cover for healing tissue. The aforementioned article by Zook, et al entitled "A study or nail bed injuries: Causes, treatments and prognosis" describes techniques using either non-adherent gauze or 0.020 inch (0.5 mm) silicone sheet. It might be assumed that the silicone sheet will have more firmness than the gauze; but even silicone sheet is so soft, supple and flexible as to provide essentially none of the advantages that have been described herein. (Silicone sheet having a thickness of 0.020 inch (0.5 mm) may be thought of as having the firmness or "body" of the leather in a woman's kid glove; that is, such silicone sheet is very flexible). An appropriate analogy might be to categorize Applicant's splint 10 as equivalent to a lobster's shell, while bandages and coverings of the prior art have had the flexibility of a cotton T-shirt. In fact, the splint 10 disclosed herein could even be utilized by the patient in scratching some skin that itches, if the patient should momentarily forget his injury and reach out to touch some spot--without remembering that he has been cautioned by his doctor against putting too much strain on the temporarily installed splint. The classical scratching process can also occur in what amounts to an inadvertent manner, if the patient is almost asleep and reaches out to touch some skin that is being irritated by a housefly or other insect. But as long as the sutures (which will usually be employed to anchor the distal end of the splint) can survive a scratching action, then the entire device will surely survive, because the curved sheet 12 has surprisingly strong properties.

In addition to its inherent strength, the splint's material and shape provide an advantageous spring constant. Three separate tests on a prototype of the splint have revealed that it has a spring rate of about 21 pounds per inch (375 kg/m) when loaded vertically, as indicated by the symbol $L_T$ in Figures 2 and 3, with the ends 16, 18 being unconstrained--as they would be when the splint is being worn by a patient. That is, the soft tissue of the eponychial fold will "anchor" the promximal end 18 of the splint 10 to the extent that it prevents the splint from moving vertically away from a digit; but the fold is too soft to prevent some longitudinal movement of the splint if the splint is subjected to loading in the direction indicated by $L_T$.

A distinguishing feature of the sheet 12 in this device is the gently concave and centrally unobstruced surface on the bottom of the splint 10, i.e., in the region under the splint that is adapted to contact the exposed tissue of the nail bed. This smooth, unobstructed surface contributes to an appearance that is markedly different from some earlier devices that have been disclosed by this inventor--in that there is no depending protuberance in the centre of the sheet which could inhibit the smooth rehabilitation of the nail bed. In one sense, the splint 10 may accurately be considered to be like a template or mould for the reason that the splint causes the healing tissue to take on the Shape of the lower surface of the splint. But this characteristic is true only if the splint 10 is held securely against the top of the healing tissue. This is more nearly assured with the preferred material described elsewhere herein, because a few common sutures can be utilized to tie the distal end of the splint to the end of the digit. That is, a surgical needle will readily penetrate a sheet of 0.020 inch (0.5 mm) polypropylene, and the small hole created by the needle will not usually tear as a result of routine physical activity by the patient. So, with the distal end 16 secured by sutures and the proximal end 18 embedded within the eponychial fold, the splint 10 will usually remain on the digit for as long as the surgeon desires.

A skirt 20 depends from each side of the sheet 12 to engage the tissue of the nail bed so as to hold the tissue and inhibit it from contracting significantly during healing, whereby the healing nail bed is restricted to taking on the smooth shape of the juxtaposed sheet as it heals. In this way, the healed nail bed has a size that tends to be almost as large as its pre-injured size.

Somewhat greater assurance against unwanted longitudinal movement of an installed splint can be obtained by use of a configuration like that shown in Figure 4, wherein the edge 22 of the depending skirt or side 20 is non-linear, e.g., shaped like a sine wave. A saw-tooth configuration for the side edge would also be feasible, as would a square wave configuration; but it is believed that the sine wave configuration is likely to be more comfortable for the patient.

If the surgeon decides to leave the splint 10 on an injured digit for several days or even a few weeks, the splint--being impervious to liquids--conceivably could trap unwanted liquids under the splint. For this reason the splint 10 has one or more "bleed" holes in its central region, so that liquids can drain spontaneously from under the splint. Four such holes 24 are shown in Figure 5. In general, a large number of small holes (no larger than 1/16 inch (1.6 mm) diameter) are preferable to a small number of larger holes.

It should be apparent from the foregoing that the splint disclosed herein is simultaneously accomplishing three separate functions: it is shaping the top of the nail bed as the bed heals; it is holding the sides of the nail bed apart, so that they do not unduly contract during healing; and it is keeping the eponychial fold intact, by preventing the fold from collapsing. However, it would be entirely feasible to accomplish one or more of these functions with elements that are not integrally formed--as is splint 10. Thus, the specific structures shown herein are intended to be exemplary and are not meant to be limiting.

## Claims

1. A temporary cover for use on a digit of a person's hand or foot in order to foster beneficial healing promptly after a nail plate has been removed, comprising:

   a) a generally elongated sheet (12) of thin but substantially rigid plastic material which is generally rectangular when seen in a plan view, and said sheet being inert to body fluids and having a curvature so that it approximates the natural shape of a human nail plate, and said sheet having a first radius which defines a curve in a longitudinal plane and a second radius which defines a curve in a transverse plane, and the transverse radius being appreciably less than the longitudinal radius, and the width of the sheet between its two sides corresponding to approximately a 70 degree segment of the circle defined by the transverse radius, and the sheet having a smooth, concave and substantially uninterrupted lower surface that is adapted for being installed so that it is in contact with and covers the nail bed, and the sheet having a proximal end (18) that is approximately perpendicular to the sides of the said sheet, whereby the proximal end of the sheet is adapted to fit under the eponychial fold of the person's digit for the purpose of preserving said fold during the healing of the nail bed, and the sheet (12) having a plurality of apertures (24) that are sized and located in order to permit the spontaneous drainage of liquid from under the sheet during the body's healing process; and

b) means depending from the two sides of said sheet (12) for engaging the tissue of the person's nail bed so as to hold said tissue and inhibit it from contracting significantly during healing of said nail bed, whereby the healing nail bed is restricted to taking on the smooth shape of the juxtaposed sheet as it heals, and whereby a healed nail bed has a size that tends to be almost as large as its pre-injured size, and whereby a new nail plate will tend to be as smooth as the sheet when the nail plate progressively grows underneath the sheet and over the healing nail bed.

2. A temporary cover according to Claim 1, characterised in that said means for engaging the tissue of the nail bed constitutes a depending skirt (20) which extends for essentially the full length of each of the two sides of the sheet (12).

3. A temporary cover according to Claim 2, characterised in that the bottom edges of said skirts are non-linear

4. A temporary cover according to any preceding Claim, characterised in that the corners between the proximal end (18) of the sheet (12) and the sides of the sheet (12) have an included angle of approximately 90 degrees.

5. A temporary cover according to any preceding claim, characterised in that the apertures (24) are located centrally on the sheet and in that said apertures (24) are no greater than about 1/16 inch (1.6 mm) in diameter, whereby the healing nail bed is restrained over nearly all of its upper surface.

6. A temporary cover according to any preceding Claim, characterised in that said sheet (12) has a thickness of approximtely 0.020 inch (0.5 mm), and wherein the longitudinal radius (R1) is about 1.5 inches (38 mm) and the transverse radius (R2) is about 0.4 inch (10 mm).

7. A temporary cover according to any preceding Claim, characterised in that the sheet (12) has an initial length of about 1 inch (25 mm), whereby there is adequate length to permit significant trimming during the process of shaping the distal end (16) of the sheet (12) to match the end of a particular digit.

**Revendications**

1. Cache temporaire à utiliser sur un doigt d'une main ou d'un pied d'une personne pour favoriser une bonne cicatrisation rapidement après qu'un ongle ait été enlevé, comprenant:

a. une lame (12) généralement allongée en une matière plastique mince mais substantiellement rigide, qui est de forme générale rectangulaire lorsqu'elle est vue en plan, et cette lame étant inerte par rapport aux liquides du corps et présentant une courbure telle qu'elle se rapproche de la forme naturelle d'un ongle humain, et ladite lame ayant un premier rayon de courbure qui définit une courbe dans un plan longitudinal et un second rayon de courbure qui définit une courbe dans un plan transversal, et le rayon de courbure transversal étant nettement plus petit que le rayon de courbure longitudinal, et la largeur de la lame entre ses deux bords latéraux correspondant à un segment d'environ 70 degrés de la circonférence définie par le rayon de courbure transversal, et la lame ayant une surface inférieure lisse, concave et substantiellement ininterrompue qui peut être posée de façon à toucher et à couvrir le tissu sous l'ongle, et la lame ayant une extrémité proximale (18) qui est approximativement perpendiculaire aux bords latéraux de ladite lame, l'extrémité proximale de la lame pouvant s'ajuster sous le repli éponychial du doigt de la personne dans le but de protéger ledit repli pendant la cicatrisation du

5

tissu sous l'ongle, et la lame (12) ayant une pluralité d'ouvertures (24) qui sont dimensionnées et localisées afin de permettre le drainage spontané du liquide provenant de sous la lame pendant le processus de cicatrisation; et

b. des moyens suspendus aux deux bords latéraux de ladite lame (12) pour s'appliquer sur le tissu sous l'ongle de la personne de façon à soutenir ledit tissu et l'empêcher de se contracter de façon importante pendant la cicatrisation dudit tissu sous l'ongle, le tissu à cicatriser sous l'ongle étant resserré pour épouser la forme lisse de la lame juxtaposée pendant qu'il se cicatrise, et le tissu cicatrisé ayant une taille qui tend à être pratiquement aussi grande que sa taille avant la blessure, et un nouvel ongle tendant à être aussi lisse que la lame lorsque l'ongle croît progressivement sous la lame et sur le tissu qui se cicatrise.

2. Cache temporaire suivant la revendication 1, caractérisée en ce que lesdits moyens à appliquer sur le tissu sous l'ongle forment une jupe (20) suspendue qui s'étend sur essentiellement toute la longueur de chacun des bords latéraux de la lame (12).

3. Cache temporaire suivant la revendication 2, caractérisée en ce que les bords inférieurs desdites jupes ne sont pas rectilignes.

4. Cache temporaire suivant l'une ou l'autre des revendications précédentes, caractérisée en ce que les coins entre l'extrémité proximale (18) de la lame (12) et les bords latéraux de la lame (12) forment un angle intérieur d'environ 90 degrés.

5. Cache temporaire suivant l'une ou l'autre des revendications précédentes, caractérisée en ce que les ouvertures (24) sont localisées en position centrale sur la lame (12) et en ce que lesdites ouvertures (24) ont un diamètre qui n'est pas supérieur à 1,6 mm (1/16 pouce), le tissu à cicatriser sous l'ongle étant ainsi soutenu sur presque la totalité de sa surface supérieure.

6. Cache temporaire suivant l'une ou l'autre des revendications précédentes, caractérisée en ce que ladite lame (12) a une épaisseur d'environ 0,5 mm (0,020 pouce), et dàns lequel le rayon de courbure longitudinal ($R_1$) est d'environ 38 mm (1,5 pouce) et le rayon de courbure transversal ($R_2$) est d'environ 10 mm (0,4 pouce).

7. Cache temporaire suivant l'une ou l'autre des revendications précédentes, caractérisée en ce que la lame (12) a une longueur initiale d'environ 25 mm (1 pouce), la longueur étant suffisante pour permettre une découpe appropriée pendant l'opération de mise en forme de l'extrémité distale (16) de la lame (12) pour la faire correspondre à l'extrémité d'un doigt particulier.

**Patentansprüche**

1. Vorübergehende Abdeckung zum Einsatz an einem Finger oder einer Zehe einer Person zur Förderung eines positiven Heilungsprozesses unmittelbar nach der Entfernung einer Nagelplatte, umfassend:

a) ein allgemein längliches Blatt (12) aus dünnem, aber im wesentlichen steifem Kunststoffmaterial, das in Draufsicht gesehen allgemein rechteckig ist, wobei jenes Blatt gegenüber Körperflüssigkeiten inert ist und eine solche Krümmung hat, daß es ungefähr die natürliche Form einer menschlichen Nagelplatte aufweist, sowie einen eine Krümmung in einer Längsebene festlegenden ersten Radius und einen eine Krümmung in einer Querebene festlegenden zweiten Radius aufweist, wobei der Querradius recht viel kleiner ist als der Längsradius und die Breite des Blattes zwischen seinen beiden Seiten ungefähr einem 70-Grad-Segment des durch den Querradius festgelegten Kreises entspricht, wobei das Blatt eine glatte, konkave und im wesentlichen ununterbrochene untere Fläche aufweist, die so angelegt werden kann, daß sie das Nagelbett berührt und abdeckt, und das Blatt ein ungefähr senkrecht zu den Seiten jenes Blattes angeordnetes proximales Ende (18) aufweist, wodurch das proximale Ende des Blattes unter die Eponychiumfalte des Fingers bzw. der Zehe der Person paßt, so daß diese Falte während des Heilens des Nagelbettes erhalten wird, wobei das Blatt (12) eine Vielzahl von Öffnungen (24) aufweist, die so groß und so angeordnet sind, daß sie die spontane Dränage von Flüssigkeit unter dem Blatt heraus während des Heilungsprozesses des Körpers gestatten; und

b) ein von den beiden Seiten jenes Blattes (12) herabhängendes Mittel, das das Nagelbettgewebe der Person so in Eingriff nimmt, daß jenes Gewebe festgehalten und daran gehindert wird, sich während der Heilung jenes Nagelbettes wesentlich zusammenzuziehen, wodurch das verheilende Nagelbett beim Heilen so eingeschränkt wird, daß es die glatte Form des daneben liegenden Blattes annimmt, und wodurch ein verheiltes Nagelbett die Tendenz hat, beinahe so groß wie vor der Verletzung zu sein, und eine neue Nagelplatte die Tendenz hat, genauso glatt wie das Blatt zu sein, wenn die Nagelplatte nach und nach unter dem Blatt und über das verheilende Nagelbett wächst.

2. Vorübergehende Abdeckung nach Anspruch 1, dadurch gekennzeichnet, daß jenes Mittel zur Ineingriffnahme des Nagelbettgewebes aus einem herabhängenden Rand (20) besteht, der sich im wesentlichen entlang der gesamten Länge jeder der beiden Seiten des Blattes (12) erstreckt.

3. Vorübergehende Abdeckung nach Anspruch 2, dadurch gekennzeichnet, daß die Unterkanten jener Ränder nicht linear sind.

4. Vorübergehende Abdeckung nach einem der vorhergehenden Ansprüche, dadurch gekennzeichnet, daß die Ecken zwischen dem proximalen Ende (18) des Blattes (12) und den Seiten des Blattes (12) einen Winkel von ungefähr 90 Grad einschließen.

5. Vorübergehende Abdeckung nach einem der vorhergehenden Ansprüche, dadurch gekennzeichnet, daß die Öffnungen (24) zentral auf dem Blatt angeordnet sind und daß der Durchmesser jener Öffnungen (24) nicht größer als etwa 1,6 mm (1/16 Zoll) ist, wodurch das verheilende Nagelbett über fast seine gesamte Oberfläche eingeschränkt ist.

6. Vorübergehende Abdeckung nach einem der vorhergehenden Ansprüche, dadurch gekennzeichnet, daß jenes Blatt (12) ungefähr 0,5 mm (0,020 Zoll) dick ist und der Längsradius (R1) etwa 38 mm (1,5 Zoll) und der Querradius (R2) etwa 10 mm (0,4 Zoll) betragen.

7. Vorübergehende Abdeckung nach einem der vorhergehenden Ansprüche, dadurch gekennzeichnet, daß das Blatt (12) eine anfängliche Länge von etwa 25 mm (1 Zoll) aufweist, wodurch eine angemessene Länge vorliegt, die wesentliches Zurechtschneiden während des Formgebungsprozesses des distalen Endes (16) des Blattes (12) zur Anpassung an das Ende eines bestimmten Fingers bzw. einer bestimmten Zehe gestattet.

FIG. 1

FIG. 2

FIG. 3

FIG. 4

FIG. 5